# EUROPEAN PATENT APPLICATION

(11) **EP 4 163 366 A1**
(43) Date of publication of application: **12.04.2023**
(21) Application number: 21820969.0
(22) Date of filing: 07.06.2021
(51) Int. Cl.: C12N 5/0783, C07K 14/54

(54) **GENETICALLY-MODIFIED CELL LINE FOR NK CELL ACTIVATION AND AMPLIFICATION, AND USE THEREOF**

(30) Priority: 09.06.2020 KR 20200069854; 08.04.2021 KR 20210046107
(71) Applicant: Samsung Life Public Welfare Foundation, Seoul 04348 (KR); Seoul National University R & DB Foundation, Seoul 08826 (KR)
(72) Inventor: CHO, Duck, Seoul 06603 (KR); DOH, Junsang, Seoul 06597 (KR); PHAN, Thi Minh-Trang, Seoul 05667 (KR); KIM, Jinho, Seoul 06342 (KR)
(74) Representative: Patentanwälte Olbricht Buchhold Keulertz
(86) International application number: PCT/KR2021/007091
(87) International publication number: WO 2021/251707

(57) **Abstract**

The present disclosure relates to a genetically engineered cell for activation of NK cells, in which the genetically engineered cell for activation of NK cells according to an aspect may synergistically induce proliferation and activation of NK cells from a sample, and thus, there is an effect in which a method of proliferating NK cells, or NK cells proliferated thereby, may be useful as an antibody therapeutic agent or the like.

## Description

### TECHNICAL FIELD

This application claims priority to Korean Patent Application No. 1 0-2020-0069854 filed on June 9, 2020 and Korean Patent Application No. 10-2021-0046107 filed on April 8, 2021, the disclosures of which are incorporated herein in their entireties by reference.

The present disclosure relates to a genetically modified cell line for NK cell activation and expansion and uses thereof, specifically, a method of proliferating NK cells using the same.

### BACKGROUND ART

Natural killer (NK) cells are a type of cytotoxic lymphocyte important for innate immunity. NK cells react to virus-infected cells or cancer cells, and the reactions depend on the signals emitted by various activating receptors and inhibitory receptors of NK cells by reacting with their respective ligands in a target cell. Typically, when an activation signal is stronger than an inhibitory signal, NK cells can attack the target cell, but when the inhibitory signal is stronger, the NK cells do not attack. Therefore, a normal cell is not attacked by NK cells due to the presence of a ligand (major histocompatibility complex, MHC) for an inhibitory receptor of NK cells, which emits an inhibitory signal.

Some cancer cells may have a decreased number of MHCs due to the occurrence of abnormalities in MHC on the cell surface. In this case, there is no inhibitory signal for the NK cells, and the NK cells attack the target cells. The NK cells have cytotoxicity in that they secrete perforin to puncture the cell membrane of infected cells or cancer cells, and release granzyme to kill these cells. Defects in the number or anticancer activity of NK cells are being found in B-cell lymphoma patients and many other cancer patients, and it is known that NK cell dysfunction is closely related to the occurrence of these cancers.

Therefore, a treatment for cancer patients utilizing the anticancer power of such NK cells is emerging, and it is important to develop an expansion method to secure a large number of high-performance NK cells. In addition, people with reduced or abnormal function of NK cells are known to be prone to cancer and various other diseases, and thus the development of a method of measuring NK cell activity is needed.

The previously reported methods of proliferating NK cells are methods using various expensive cytokines at high concentrations or using peripheral blood mononuclear cells or cancer cell lines together. However, this requires high costs and the expansion rate is not very high. In addition, in many studies, expansions of other lymphocytes, such as T cells, are accompanied by NK cell expansion, making the method unsuitable for NK cell-based immune cell therapy. Therefore, the development of an efficient method for selectively amplifying only NK cells is a major subject of the task, and research is being conducted (Korean Patent No. 10-1525199), but is still incomplete.

### DESCRIPTION OF EMBODIMENTS

### TECHNICAL PROBLEM

An aspect is to provide a feeder cell, or a cell line genetically engineered to express membrane-bound interleukin-18 (mblL-18), membrane-bound interleukin-21 (mbIL-21), and/or OX40L.

Another aspect provides a method of proliferating NK cells including obtaining a blood sample containing a mixed NK cell population (mixed immune cells); and contacting at least a part of the NK cell population with a genetically engineered cell in order to activate the NK cells, wherein the genetically engineered cell is genetically engineered to express membrane-bound interleukin-18 (mblL-18), membrane-bound interleukin-21 (mbIL-21), and/or OX40L.

### SOLUTION TO PROBLEM

An aspect provides a cell line genetically engineered to express membrane-bound interleukin-18 (mblL-18), membrane-bound interleukin-21 (mbIL-21), and/or OX40L. In an embodiment, the cell line may be genetically engineered to express mblL-18, mblL-21, and OX40L.

The cell line may be used as feeder cells for selectively amplifying only NK cells. In general, a K562 cell line, which is representative feeder cells used for NK cell proliferation, is only used for proliferating NK cells, and the K562 cell line itself is not to be proliferated. Therefore, before culturing with NK cells, the K562 cell line is pre-treated by irradiation with strong radiation (for example, 50 to 100 Gy), in order that the K562 cell line itself does not proliferate at all, and only aids in the proliferation of NK cells. On the other hand, most cytokines such as IL-2 and IL-15 must be continuously exposed to NK cells for the efficient proliferation of NK cells. Therefore, when a cancer cell line-based feeder cell is genetically engineered to express cytokines such as IL-2 and/or IL-15 since the cancer cell line pre-treated with radiation cannot survive for a long time in the culturing process, there is an issue of low efficiency of the selective proliferation of NK cells. In an embodiment, when NK cells were exposed to IL-18 or IL-21 for a short period in the presence of K562-OX40L, the cell amplification effect did not appear until the 21st day, but when simultaneously exposed to IL-18 and IL-21, the cell amplification effect was found to appear after 28 days. Therefore, IL-18 and IL-21 were effective in proliferating NK cells despite only one exposure and a short-term exposure on the first day of culture. That is, in the feeder cell line genetically engineered to express mbIL-18 and mbIL-21 according to an aspect, the efficiency of NK cell proliferation is not reduced despite the short-term expression of IL-18 and IL-21, and only NK cells may be selectively expanded.

The term "feeder cells (also referred to as support cells)", used herein, may refer to cells that help the proliferation of target NK cells by producing various metabolites since the cells have metabolic activity, although the cells lack an ability to divide and proliferate by being irradiated. Feeder cells that may be used in the present specification are animal cell lines into which a gene is introduced, and may be a human chronic myelogenous leukemia cell line (for example, K562 cell), RPMI8866, EBV_LCL, 721.221, HFWT, NK-92, etc.

The term "genetic engineering" or "genetically engineered", used herein, refers to an act of introducing at least one genetic modification into a cell, or to cells thereby created.

Specifically, the genetically engineered cell may include an exogenous gene encoding the above-mentioned gene. The term "exogenous" means that a referenced molecule or a referenced activity is introduced into a host cell. A molecule may be inserted into the host chromosome, as, for example, an encoding nucleic acid that is introduced into the host genetic material, or as a non-chromosomal genetic material such as a plasmid. With respect to the expression of an encoding nucleic acid, the term "exogenous" indicates that the encoding nucleic acid is introduced into a subject in a form that is expressible. With respect to the expression of an encoding nucleic acid, the term "exogenous" refers to an activity introduced into a host parent cell. The source of the biosynthetic activity may be, for example, a homologous or heterologous encoding nucleic acid that expresses a referenced activity after being introduced into a host parent cell. Thus, the term "endogenous" refers to the referenced molecule or activity present in the host cell. Similarly, with respect to the expression of an encoding nucleic acid, the term "endogenous" refers to the expression of the encoding nucleic acid included in a subject. The term "heterologous" refers to a molecule or activity from an origin other than the referenced species and the term "homologous" refers to a molecule or activity from the host parent cell. Thus, exogenous expression of an encoding nucleic acid may utilize any one or both of heterologous or homologous encoding nucleic acids.

Accordingly, the cell may include a nucleic acid encoding mblL-18, mblL-21, and/or OX40L. More specifically, the cell may be one transformed with a vector including a nucleic acid encoding mblL-18, mblL-21, and/or OX40L.

The term "vector", used herein, refers to a vector capable of expressing a target protein in a suitable host cell and refers to a genetic construct including regulatory elements operably linked to express a gene insert. A vector according to an embodiment may include regulatory elements for expression such as a promoter, an operator, a start codon, a stop codon, a polyadenylation signal, and/or an enhancer, and the promoter of the vector may be constitutive or inducible. In addition, the vector may be an expression vector capable of stably expressing the fusion protein in a host cell. The expression vector may be a vector that is used in the related art to express an exogenous protein in plants, animals or microorganisms. The recombinant vector may be constructed by using various methods known in the art. For example, the vector may include a selectable marker for selecting host cells to include the vector, and in the case of a replicable vector, it may include an origin of replication. In addition, the vector may be self-replicating or introduced into host DNA, and the vector may be one selected from the group consisting of a plasmid, a lentivirus, an adenovirus, an adeno-associated virus, a retrovirus, a herpes simplex virus, and a vaccinia virus.

In addition, in the vector, the polynucleotide sequence encoding the above-described fusion protein may be operably linked to a promoter. The term "operably-linked", used herein, refers to a functional linkage between a regulatory sequence for the expression of nucleic acid (for example, a promoter, a signal sequence, or an array of binding sites of transcriptional regulatory elements) and another nucleic acid sequence, whereby the regulatory sequence regulates the transcription and/or translation of the other nucleic acid sequence.

The term "membrane-bound interleukin", used herein, refers to an interleukin bound to a cell membrane and may have a meaning distinct from an interleukin that is secreted extracellularly. mbIL-18 may have sequence homology with a nucleic acid sequence of SEQ ID NO: 1 or an amino acid sequence thereof of about 70 % or more, about 75 % or more, about 80 % or more, about 85 % or more, about 90 % or more, about 92 % or more, about 95 % or more, about 97% or more, about 98% or more, or about 99% or more. mbIL-21 may have sequence homology with a nucleic acid sequence of SEQ ID NO: 2 or an amino acid sequence thereof of about 70 % or more, about 75 % or more, about 80 % or more, about 85 % or more, about 90 % or more, about 92 % or more, about 95 % or more, about 97% or more, about 98% or more, or about 99% or more.

The term "OX40L", used herein, is a ligand of OX40 (CD134), which is a member of the TNF family and is known to be expressed on antigen-presenting cells (APCs) such as B cells, macrophages, endothelial cells, and dendritic cells (DC). OX40L may be one having sequence homology with a nucleic acid sequence of SEQ ID NO: 3 or an amino acid sequence thereof of about 70 % or more, about 75 % or more, about 80 % or more, about 85 % or more, about 90 % or more, about 92 % or more, about 95 % or more, about 97% or more, about 98% or more, or about 99% or more.

Another aspect provides a composition for culturing NK cells including cells genetically engineered to express membrane-bound interleukin-18 (mblL-18), membrane-bound interleukin-21 (mbIL-21), and/or OX40L.

Specific details of the genetically engineered cells are described above.

The composition for culturing NK cells including cells genetically engineered to express mblL-18, mblL-21, and OX40L according to an embodiment may induce expansion and/or activation of NK cells (for example, natural killer cells), and thus, may be useful for culture, isolation, or proliferation of NK cells.

Another aspect provides a method of proliferating NK cells including obtaining a blood sample containing a population of mixed NK cells (mixed immune cells); and contacting at least a part of the mixed NK cell population with a genetically engineered cell in order to activate the NK cells, wherein the genetically engineered cell is genetically engineered to express membrane-bound interleukin-18 (mblL-18), membrane-bound interleukin-21 (mbIL-21), and/or OX40L.

In an embodiment, the contact may include co-culturing the genetically engineered cells and the mixed NK cell population to stimulate, activate, or expand a subpopulation of the NK cells.

The term "stimulation of NK cells", used herein, may refer to increased activity of natural killer cells, for example, cytotoxic activity, in vitro or in vivo, or generating, increasing, amplifying, or proliferating activated natural killer cells.

In an embodiment, non-limiting examples of NK cells include macrophages, B lymphocytes, T lymphocytes, mast cells, monocytes, dendritic cells, eosinophils, natural killer cells, basophils, and neutrophils. Accordingly, in certain embodiments, the NK cell may be any one selected from the group consisting of macrophages, B lymphocytes, T lymphocytes (CD8+ CTL), mast cells, monocytes, dendritic cells, eosinophils, natural killer cells, basophils, and neutrophils. In certain embodiments, the NK cells may be natural killer cells or T lymphocytes. The mixed NK cells may include one or more selected from the group consisting of macrophages, B lymphocytes, T lymphocytes, mast cells, monocytes, dendritic cells, eosinophils, natural killer cells, basophils, and neutrophils.

The term "natural killer cells" or "NK cells", used herein, refers to cytotoxic lymphocytes constituting a major component of the innate immune system, which are defined as large granular lymphocytes (LGL) and constitute a third cell differentiated from B and T lymphocytes generated from a common lymphoid progenitor (CLP). The "natural killer cells" or "NK cells" include natural killer cells without additional modification derived from any tissue source, and may include mature natural killer cells as well as progenitor cells of natural killer cells. The natural killer cells are activated in response to interferon or macrophage-derived cytokines, and the natural killer cells include two types of surface receptors that control the cytotoxic activity of the cells, labeled as "activating receptors" and "inhibiting receptors". Natural killer cells may be generated from any source, for example, hematopoietic cells from placental tissue, placental perfusion, umbilical cord blood, placental blood, peripheral blood, spleen, liver, etc., example, from hematopoietic stems or precursors.

In an embodiment, the natural killer cells may be activated natural killer cells. The activated natural killer cells may refer to cells in which cytotoxicity or intrinsic immunomodulatory ability of natural killer cells is activated compared to parental cells, for example, hematopoietic cells, or progenitor cells of natural killer cells. In a specific embodiment, the activated natural killer cell is CD3-CD56+.

In an embodiment, activated natural killer cells or a population enriched with activated natural killer cells may be evaluated by detecting at least one kind of related markers, for example, CD16, CD57, CD69, CD94, CD161, CD158a, CD158b, NKp30, NKp44, NKp46, DNAM-1, 2B4, NKp46, CD94, KIR (for example, KIR2DL1, KIR2DL2/3, KIR3DL1), and a NKG2 family of an activating receptor (for example, NKG2A, NKG2C, NKG2D).

In an embodiment, co-culturing may be performed in the presence of cytokines.

The term "cytokine", used herein, may refer to a protein (about 5 kDa to 20 kDa) that plays a role in cell signaling. Cytokines are released by cells and affect the behavior of cytokine-releasing cells and/or other cells. Non-limiting examples of cytokines include chemokines, interferons, interleukins, lymphokines, tumor necrosis factors, monokines, and colony-stimulating factors. Cytokines may be produced by a wide range of cells including (but not limited to) immune cells such as macrophages, B lymphocytes, T lymphocytes, mast cells and monocytes, endothelial cells, fibroblasts, and interstitial cells. Cytokines may be produced by one or more types of cells. Cytokines act through receptors and are of particular importance in the immune system, regulate the balance between humoral and cellular immune responses, and regulate the maturation, growth, and responsiveness of cell populations. A cytokine herein may be a naturally occurring cytokine or a mutated version of a naturally occurring cytokine. "Naturally occurring", used herein, may also be referred to as the wild-type and includes allelic variants. A mutated version or a "mutant" of a naturally occurring cytokine refers to specific mutants derived from the naturally occurring sequence to alter the function, activity and/or specificity of the cytokine. In an embodiment, the mutants may enhance the function, activity and/or specificity of the cytokine. In another embodiment, the mutants may reduce the function, activity and/or specificity of a cytokine. Mutants may include deletions or additions of one or more amino acid residues of the cytokine.

The cytokine may be any one selected from the group consisting of a bone morphogenetic protein (BMP) family, a chemokine ligands (CCL) family, a CKLF-like MARVEL transmembrane domain containing member (CMTM) family, a C-X-C motif ligand (CXCL) family, a growth/differentiation factor (GDF) family, a growth hormone, an interferon (IFN) family, an interleukin (IL) family, a tumor necrosis factors (TNF) family, glycophosphatidylinositol (GPI), secreted *Ly-6*/*uPAR-Related* Protein 1 (SLUPR-1), secreted *Ly-6*/*uPAR-Related* Protein 2 (SLUPR-2), and a combination thereof.

In an embodiment, the cytokine is interleukin or a mutant thereof. Many interleukins are synthesized by helper CD4 T lymphocytes, as well as monocytes, macrophages, and endothelial cells. Interleukins are capable of promoting the development and differentiation of T and B lymphocytes and hematopoietic cells. Non-limiting examples of interleukins include IL1, IL2, IL3, IL4, IL5, IL6, IL7, IL8 (CXCL8), IL9, IL10, IL11, IL12, IL13, IL14, IL15, IL16, IL17, IL18, IL19, IL20, IL21, IL22, IL23, IL24, IL25, IL26, IL27, IL28, IL29, IL30, IL31, IL32, IL33, IL35, or IL36. Thus, in certain embodiments, the cytokine may be interleukin or a mutant thereof, including wild-type and mutant forms of IL1, IL2, IL3, IL4, IL5, IL6, IL7, IL8 (CXCL8), IL9, IL10, IL11, IL12, IL13, IL14, IL15, IL16, IL17, IL18 , IL19, IL20, IL21, IL22, IL23, IL24, IL25, IL26, IL27, IL28, IL29, IL30, IL31, IL32, IL33, IL35, or IL36.

In another embodiment, the cytokines added to the co-culture medium may be IL-18 and IL-21. More specifically, the co-culturing may include adding IL-18 and IL-21 to the culture medium while culturing in the presence of IL-2 and IL-15. The IL-21 may be used at a concentration of about 1 ng/ml to about 20 ng/ml, about 1 ng/ml to about 15 ng/ml, about 1 ng/ml to about 10 ng/ml, or about 2 ng/ml to about 8 ng/ml. The IL-18 may be used at a concentration of about 10 ng/ml to about 200 ng/ml, about 10 ng/ml to about 180 ng/ml, about 20 ng/ml to about 160 ng/ml, about 20 ng/ml to about 120 ng/ml, about 20 ng/ml to about 40 ng/ml, about 20 ng/ml to about 80 ng/ml, about 40 ng/ml to about 160 ng/ml, about 40 ng/ml to about 120 ng/ml, about 40 ng/ml to about 80 ng/ml, about 80 ng/ml to about 160 ng/ml, or about 80 ng/ml to about 120 ng/ml. In an embodiment, by exposing the cells to IL-18 and IL-21 during co-culturing, the proliferation of NK cells may be more synergistically increased.

In an embodiment, the co-culturing may be co-culturing for 2 to 30 days.

In an embodiment, the genetically engineered cells may be treated with radiation of 50 Gy to 300 Gy.

In an embodiment, the sample may be a biological sample derived from a subject, for example, a mammal including a human. In addition, the biological sample may be one isolated from a subject and maybe blood, whole blood, serum, plasma, lymph, urine, feces, tissue, cell, organ, bone marrow, saliva, sputum, cerebrospinal fluid, or a combination thereof. In addition, the biological sample may include peripheral blood mononuclear cells (PBMCs), purified NK cells, or primary resting cells (that is, directly isolated from blood).

Another aspect provides NK cells produced by the method of proliferating NK cells.

Another aspect provides a cell therapy product including the immune cell or a cell population thereof as an active ingredient.

Another aspect provides a pharmaceutical composition for preventing or treating cancer or infectious diseases using the immune cells or the cell population thereof as an active ingredient.

Another aspect provides the use of the immune cell or cell population thereof for the manufacture of medicine.

Another aspect provides a method of treating disease including administering the immune cell or the cell population thereof to a subject.

The term "disease", used herein, may refer to a pathological condition, particularly, cancer, an infectious disease, an inflammatory disease, a metabolic disease, an autoimmune disease, a degenerative disease, a disease related to apoptosis, and graft rejection.

The term "treatment", used herein, may refer to or includes alleviation, inhibition of progression, or prevention of a disease, disorder or condition, or one or more symptoms thereof, and "active ingredient" or "pharmaceutically effective amount" means any amount of a composition used in the practice of the disclosure provided herein, which is sufficient to alleviate, inhibit the progression, or prevent a disease, disorder or condition, or one or more symptoms thereof.

The terms "administering", "introducing", and "implanting", used herein, are used interchangeably, and may refer to the placement of a composition according to an embodiment into a subject by a method or a route that results in at least partial localization of the composition to the desired site. A composition according to an embodiment may be administered by any suitable route that delivers at least a portion of a cell or a cellular component to the desired location in a living subject. A survival period of the cells after being administered to a subject may be as short as several hours, for example, from 24 hours to several days, or as long as several years.

The term "isolated cell", for example, "isolated immune cell" or the like refers to a cell substantially isolated from a tissue from which the cell originates, for example, a hematopoietic cell.

A method of administering the pharmaceutical composition according to an embodiment is not particularly limited, but the pharmaceutical composition may be orally administered or parenterally administered, for example, intravenously, subcutaneously, intraperitoneally, or by inhalation or topical application, depending on the desired method. A dose has a wide range according to the patient's weight, age, sex, health, diet, administration time, administration manner, excretion rate, and severity of the disease. A daily dose means a sufficient amount of the therapeutic material according to an aspect that is administered to a subject that requires treatment to alleviate symptoms of the disease. An effective amount of the therapeutic material may vary according to the specific compound, state and severity of the disease, the subject requiring treatment, and may be determined by those skilled in the art. As a non-limiting example, a dose of the composition according to an aspect of a human subject may vary according to the patient's age, body weight, sex, administration form, health, and severity of the disease. With respect to an adult patient weighing 70 kg, for example, about 1,000 cells/time to about 10,000 cells/time, about 1,000 cells/time to about 100,000 cells/time, about 1,000 cells/time to about 1000,000 cells/time, about 1,000 cells/time to about 10,000,000, about 1,000 cells/time to about 100,000,000 cells/time, about 1,000 cells/time to about 1,000,000,000 cells/time, about 1,000 cells/time to about 10,000,000,000 cells/time, may be divided and administered once to several times a day at regular time intervals, or may be administered several times at regular time intervals.

The term "subject" refers to a subject requiring treatment of a disease, or more specifically, may refer to human or non-human primates, or a mammal such as a mouse, a rat, a dog, a cat, a horse, or a cow.

The pharmaceutical composition according to an embodiment may include a pharmaceutically acceptable carrier and/or additive. For example, the pharmaceutical composition may include sterile water, physiological saline, conventional buffers (phosphoric acid, citric acid, other organic acids, etc.), stabilizers, salts, antioxidants (ascorbic acid, etc.), surfactants, suspending agents, isotonic agents, or preservatives, etc. For topical administration, organic materials such as biopolymers, inorganic materials such as hydroxyapatite, specifically, combinations with a collagen matrix, polylactic acid polymers or copolymers, polyethylene glycol polymers or copolymers and chemical derivatives thereof, etc. may also be included. When the pharmaceutical composition according to an embodiment is formulated in a formulation suitable for injection, immune cells, or substances that increase their activity may be dissolved in a pharmaceutically acceptable carrier or may be frozen as a dissolved solution.

A pharmaceutical composition according to an embodiment may contain a suspending agent, a solubilizing agent, a stabilizer, an isotonic agent, a preservative, an adsorption inhibitor, a surfactant, a diluent, an excipient, a pH adjuster, an analgesic agent, a buffer, a reducing agent, an antioxidant and the like as appropriate according to a method of administration or formulation. Pharmaceutically acceptable carriers and formulations suitable for the present disclosure, including those exemplified above, are described in detail in Remington's Pharmaceutical Sciences, 19th ed., 1995. A pharmaceutical composition according to an embodiment may be prepared in a unit dosage form formulated by using a pharmaceutically acceptable carrier and/or excipient or may be prepared by putting the composition into a multi-dose container, according to a method that may be easily carried out by a person of ordinary skill in the art to which the present disclosure pertains. In this regard, the formulation may be in a form of a solution, suspension, or emulsion in an oily or aqueous medium, or in a form of a powder, granules, tablets, or capsules.

### ADVANTAGEOUS EFFECTS OF DISCLOSURE

According to a genetically engineered cell according to an aspect, the proliferation and activity of NK cells may be increased at least 2-fold to several-fold compared to cells that are not genetically engineered, and thus, the genetically engineered cell may be used as a cell therapy product by proliferating NK cells.

### BRIEF DESCRIPTION OF DRAWINGS

FIG. 1A is a graph confirming characteristics of expression of mbIL-18 and mblL-21 in feeder cells according to an aspect.
FIG. 1B is a combined image of feeder cells according to an aspect and mIL-18 and mIL-21 (left) and a graph (right) in which the amounts of mIL-18 and mIL-21 are quantified by fluorescence intensity.
FIG. 2A is a graph showing the fold expansion of NK cells when the NK cells are exposed for a short period of time to IL-21 alone or IL-18 and IL-21, in the presence of K562-OX40L cells.
FIG. 2B is a graph showing the purity of NK cells when the NK cells are exposed for a short period of time to IL-21 alone or IL-18 and IL-21, in the presence of K562-OX40L cells.
FIG. 2C is graphs showing fold expansions of NK cells when the NK cells are exposed for a short period of time to IL-21 alone or IL-18 and IL-21, in the presence of K562-OX40L cells.
FIG. 3 is a graph comparing the expansion (left) and purity (right) of NK cells using feeder cells according to an aspect.
FIGS. 4A and 4B are graphs comparing markers expressed in healthy donor (HD)-derived NK cells expanded by using feeder cells according to an aspect.
FIG. 5A is a graph confirming cytokines expressed in a healthy donor (HD)-derived NK cells expanded by using feeder cells according to an aspect.
FIG. 5B is a graph showing the results of CD107a degranulation analysis of a healthy donor (HD)-derived NK cells expanded by using feeder cells according to an aspect.
FIG. 6A is a graph confirming the cytotoxicity of healthy donor (HD)-derived NK cells expanded by using feeder cells according to an aspect.
FIG. 6B is a graph confirming antibody-dependent cellular cytotoxicity (ADCC) of a healthy donor (HD)-derived NK cells expanded by using feeder cells according to an aspect.

### MODE OF DISCLOSURE

Hereinafter, preferred embodiments are presented to help the understanding of the present disclosure. However, the following embodiments are only provided for an easier understanding of the present disclosure, and the contents of the present disclosure are not limited by the following embodiments.

### [Examples]

### Example 1. Preparation of genetically engineered feeder cells

Feeder cells expressing membrane bound interleukin-18 (mblL-18), membrane bound interleukin-21 (mbIL-21), and/or OX40L were prepared. Specifically, a human-derived mbIL18 gene (SEQ ID NO: 1) and a human-derived mblL21 gene (SEQ ID NO: 2) were cloned into a lentiviral vector, pCDH-CMV-RFP, to prepare recombinant lentiviral production vectors. Then, the recombinant gene (pCDH-CMV-RFP-mbIL-1821) prepared above for virus production was transfected into 293FT cells by using lipofectamin3000 (Invitrogen) together with a packaging vector. After a period of time, the medium was replaced with a fresh medium and the cells were cultured for 48 hours, and then the medium containing the virus was recovered. The recovered medium was centrifuged at 500 × g for 10 minutes, and only the pure medium containing the virus was separated by using a 0.45 µm filter to produce a mbIL18-mbIL21-expressing lentivirus. Thereafter, 1 ml of the lentivirus expressing mbIL18-mbIL21 was dissolved in 9 ml of the medium containing K562-OX40L cells of Comparative Example 1 below and added with polybrene (8 µg/ml), and cell culturing was proceeded for 48 hours. Next, in order to select only infected cells, only cells expressing both green and red fluorescence were selected by using an automatic ultrafast cell sorter, and the expression of mbIL18-mbIL21 was analyzed by flow cytometry (FACS) to produce a K562 cell line expressing mblL18-mblL21 (hereinafter, "K562-OX40L-mbIL18-mbIL21 ").

### [Comparative Example]

### Comparative Example 1. Preparation of genetically engineered feeder cells

Feeder cells expressing OX40L were prepared. Specifically, a human-derived OX40L gene (SEQ ID NO: 3) was cloned into a lentiviral vector, pLVX-IRES-ZsGreen, to prepare a recombinant lentiviral production vector. Then, the recombinant gene (OX40L-pLVX-IRES-ZsGreen) was transfected into 293FT cells by using lipofectamin3000 (Invitrogen) together with a packaging vector for virus production. After a period of time, the medium was replaced with a fresh medium and the cells were cultured for 48 hours, and then the medium containing the virus was recovered. The recovered medium was centrifuged at 500 × g for 10 minutes, and only the pure medium containing the virus was separated by using a 0.45 µm filter to produce an OX40L-expressing lentivirus. Thereafter, 1 ml of the lentivirus expressing OX40L was dissolved in 9 ml of medium (RPMI 1640) containing K562 cells and added with polybrene (8 µg/ml), and cell culturing proceeded for 48 hours. Next, in order to select only infected cells, only cells expressing green fluorescence were selected by using an automatic ultrafast cell sorter, and the expression of OX40L was analyzed by flow cytometry (FACS) to produce a K562 cell line expressing OX40L (hereinafter, "K562-OX40L").

### [Experimental Examples]

### Experimental Example 1. Characteristics of genetically engineered feeder cells

In order to confirm the characteristics of expression of mbIL-18 and mbIL-21 of the genetically engineered feeder cells according to an aspect, expression levels of mRNA and surface protein were identified.

Specifically, the total RNA of the K562-OX40L-mbIL18-mbIL21 cell line prepared in Example 1 and the K562-OX40L cell line prepared in Comparative Example 1 was isolated by using an RNeasy Mini Kit (Qiagen, Venlo, Netherlands) and quantified by using an IMPLEN Nanophotometer P330 (IMPLEN, Munich, Germany). Thereafter, the isolated RNA was converted to cDNA by using a QuantiTect Reverse Transcription Kit (Qiagen), and then PCR was performed in a standard 20 µl reaction volume by using a QuantiTect SYBR Green PCR Kit (Qiagen) and Rotor-Gene Q (Qiagen). In this regard, the primers in Table 1 below were used, and all experiments were repeated three times.

**[Table 1]**

| Gene | Directio n | Primer sequence (5' to 3') | Accession No. |
|---|---|---|---|
| *IL-18* | Forward | CATTGACCAAGGAAATCGGC | NM_001562 |
| | Reverse | CACAGAGATAGTTACAGCCA TACC | |
| *IL-21* | Forward | AAGCTGAAGAGGAAACCACC | NM_021803 |
| | Reverse | TCTTTCTAGGAATTCTTTGGG TGG | |
| *GAPDH* | Forward | ACATCGCTCAGACACCATG | NM_002046 |
| | Reverse | TGTAGTTGAGGTCAATGAAG GG | |

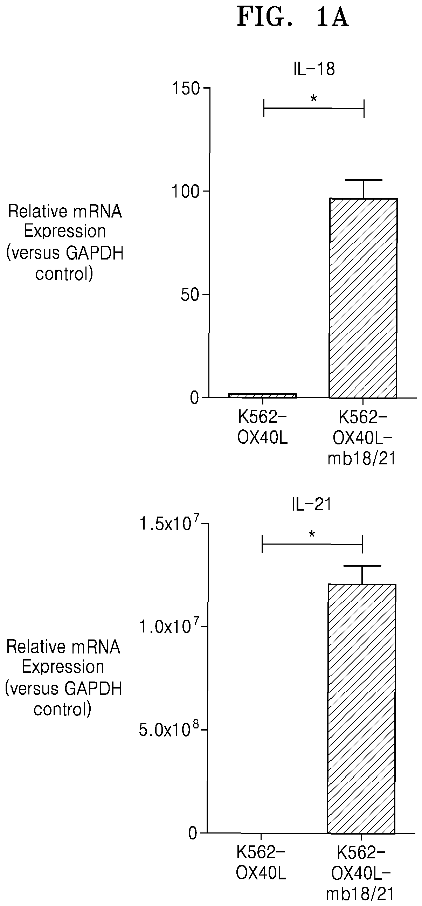

FIG. 1A is a graph confirming characteristics of expression of mbIL-18 and mblL-21 in feeder cells according to an aspect.

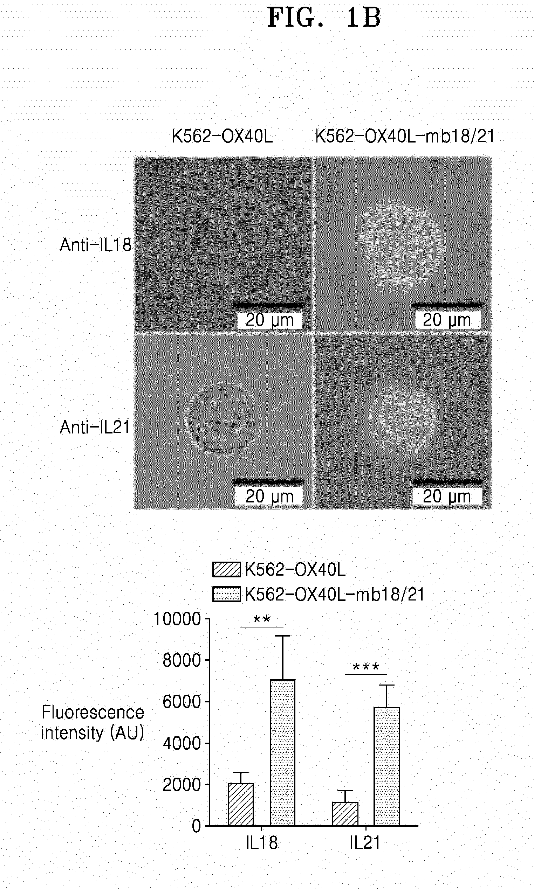

FIG. 1B is a combined image of the feeder cells according to an aspect and mlL-18 and mIL-21 (left) and a graph (right) in which the amounts of mIL-18 and mIL-21 are quantified by fluorescence intensity.

As a result, as shown in FIG. 1A, mbIL-18 and mbIL-21 mRNA were detected in the feeder cells of Embodiment 1, but mbIL-18 and mbIL-21 mRNA were not detected in the feeder cells of Comparative Example 1. In addition, as shown in FIG. 1B, it was confirmed that larger amounts of IL-18 and IL-21 were continuously expressed on the surface of the feeder cells of Embodiment 1 compared to the feeder cells of Comparative Example 1.

### Experimental Example 2. Confirmation of NK cell activation through addition of K562-OX40L cells, and IL18 and IL21

In order to predict the NK cell activation efficacy of the genetically engineered feeder cells according to an aspect, the degree of NK cell activation according to the addition of cytokines in K562-OX40L cells was confirmed in a preliminary experiment.

Specifically, the K562-OX40L cell line prepared in Comparative Example 1 was cultured in a T-25 flask containing 25 ml of complete RPMI 1640 medium (containing FBS, penicillin, and streptomycin) at 37°C in an incubator supplied with 5 % of CO₂. Thereafter, centrifugation was performed under the condition of 400 × g for 3 minutes, and the cell pellet was resuspended in 5 ml of complete RPMI 1640 medium to harvest feeder cells. Thereafter, in order to prevent excessive growth of the feeder cells, the feeder cells were irradiated with gamma rays at 100 Gy by using a Gammacell 3000 Elan radiator and used in subsequent experiments.

In order to isolate peripheral blood mononuclear cells (PBMCs), normal donor's whole blood: PBS was diluted at a ratio of 1:2 (10 ml of whole blood: 20 ml of PBS) and overlaid on a 15 ml Lymphoprep. Next, centrifugation was performed without a brake under the condition of 1200 × g for 25 minutes at room temperature (acceleration 1, deceleration 0), cells were harvested from the buffy coat layer, and washed three times with PBS while being centrifuged at 400 × g for 7 minutes. After inoculating the 3 × 10⁶ isolated peripheral blood mononuclear cells and 0.5 × 10⁶ K562-OX40L cells irradiated at 100 Gy onto a 24-well plate containing 1 ml of NK cell medium, 1 ml of NK cell medium containing 20 U/ml of IL-2 was added to make a total medium volume of 2 ml/well and a final concentration of IL-2 of 10 U/ml, and then after gently pipetting to mix, the mixture was cultured in a 5 % CO₂ incubator at 37 °C. On the 7th day of culturing, 100 U/ml of IL-2 and 5 ng/ml of IL-15 were added to the medium, and cultured for 14 days, replacing with fresh medium every 2 to 3 days. Thereafter, 5 ng/ml of IL-21, and/or 25, 50, and 100 ng/ml of IL-18 were added on day 0 of the culturing and further cultured for 14 days.

The expansion of NK cells was confirmed by using fluorescein isothiocyanate (FITC)-conjugated mouse anti-human CD 3 and PE-Cy5-conjugated mouse anti-human CD 56 monoclonal antibodies, and each fold expansion was identified with the purity of the NK cells, when treating with K562 cells and IL-2/IL-15, as a baseline.

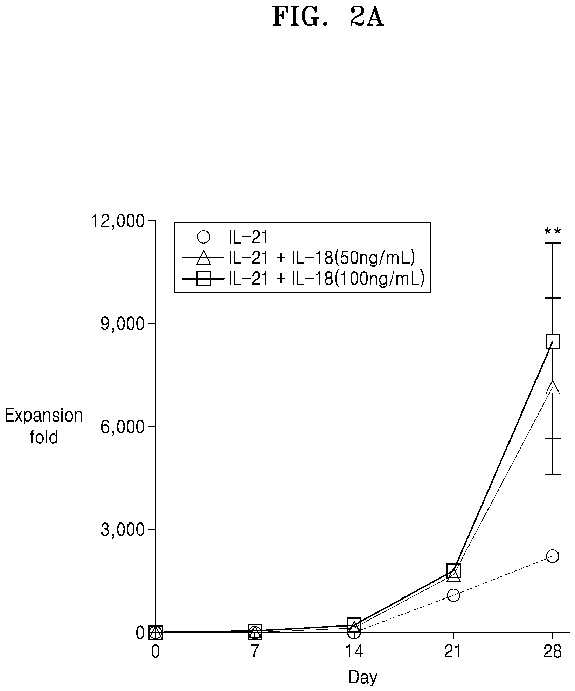

FIG. 2A is a graph showing fold expansion of NK cells when the NK cells were exposed to IL-21 alone or IL-18 and IL-21, in the presence of K562-OX40L cells for a short period of time.

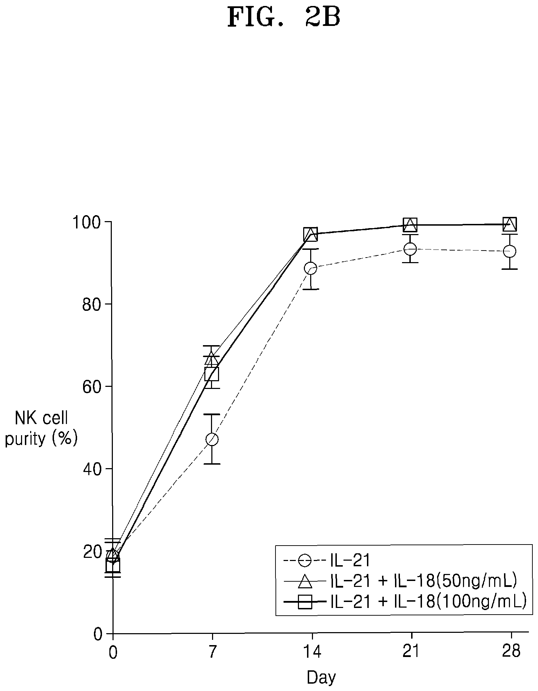

FIG. 2B is a graph showing purity of NK cells when the NK cells were exposed for a short period of time to IL-21 alone or IL-18 and IL-21, in the presence of K562-OX40L cells.

As a result, as shown in FIG. 2A, the effect of short-term exposure of NK cells to IL-18 and IL-21 was not identified until the 21st day. However, the expansion of NK cells was confirmed to be significantly increased after 28 days. In addition, as shown in FIG. 2B, NK cell purity of the NK cells exposed for a short period to both II-18 and IL-21 was confirmed to be higher than that of the NK cells treated with IL-21 only on the 28th day.

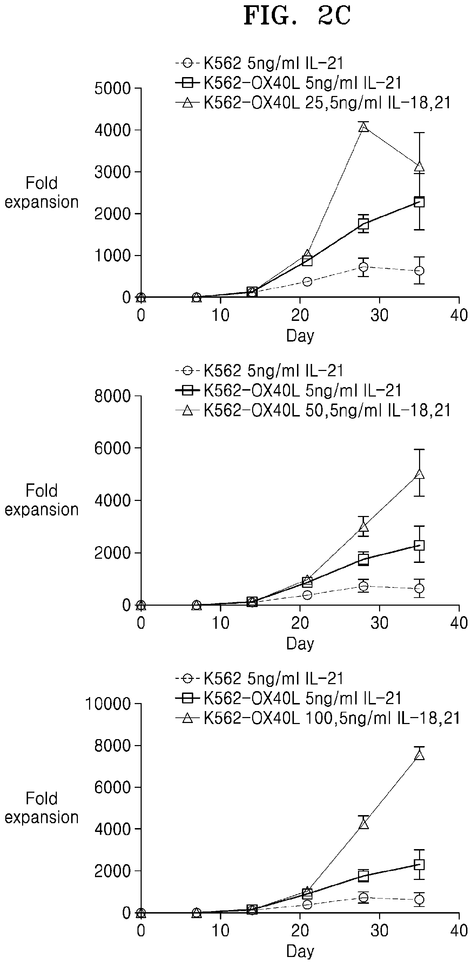

FIG. 2C is graphs showing fold expansions of NK cells when the NK cells were exposed for a short period of time to IL-21 alone or IL-18 and IL-21, in the presence of K562-OX40L cells.

As a result, as shown in FIG. 2C, activity of NK cells was confirmed to be increased by about 2 to 4 times when K562-OX40L cells were treated with both of IL-18 and IL-21, compared to when IL-18 or IL-21 was each treated.

These results indicate that OX40L-expressing cells not only significantly increase the activity of NK cells, but also synergistically increase the activity of NK cells with additional treatment of IL-18 and IL-21 for a short period of time. That is, feeder cells expressing OX40L may exhibit a synergistic effect on the activity of NK cells by treatment with IL-18 and IL-21.

### Experimental Example 3. NK cell activation and expansion by using K562-OX40L-mbIL18-mbIL21 cells

Based on the results of Experimental Example 2, the effect of K562-OX40L-mbIL18-mbIL21 cells prepared in Example 1 on activity of NK cells was identified.

Specifically, the K562-OX40L-mbIL18-mbIL21 cell line of Embodiment 1 was cultured in a T-25 flask containing 25 ml of complete RPMI 1640 medium in an incubator supplied with 5 % CO₂ at 37 °C. Thereafter, centrifugation was performed under the condition of 400 × g for 3 minutes, and the cell pellet was resuspended in 5 ml of complete RPMI 1640 medium to harvest feeder cells. Thereafter, in order to prevent excessive growth of the feeder cells, the feeder cells were irradiated with gamma rays at 100 Gy by using a Gammacell 3000 Elan radiator and used in subsequent experiments. After inoculating the 3 × 10⁶ isolated peripheral blood mononuclear cells and 0.5 × 10⁶ K562-OX40L cells irradiated at 100 Gy onto a 24-well plate containing 1 ml of NK cell medium in the same manner as in Experimental Example 2, 1 ml of NK cell medium containing 20 U/ml of IL-2 was added to make a total medium volume of 2 ml/well and a final concentration of IL-2 of 10 U/ml, and then after gently pipetting to mix, the mixture was cultured in a 5 % CO₂ incubator at 37 °C. On the 7th day of culture, 100 U/ml of IL-2 and 5 ng/ml of IL-15 were added to the medium, and cultured for 14 days, replaced with a fresh medium every 2 to 3 days. As a control group, K562 cells were used. The degree of activation of NK cells was confirmed in the same manner as in Experimental Example 2 by identifying the purity and fold expansion of NK cells.

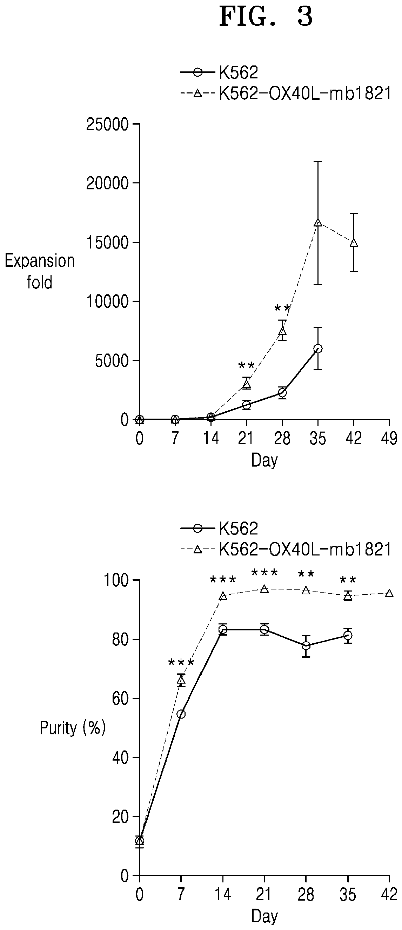

FIG. 3 is graphs comparing the expansion (left) and purity (right) of NK cells using the feeder cells according to an aspect.

As a result, as shown in FIG. 3, with the feeder cells of Embodiment 1, the fold expansion of NK cells was confirmed to be significantly increased compared to the control group. In particular, in the case of the control group, NK cells did not show any further increase after increasing until day 35 after culturing. However, Embodiment 1 showed a significant increase until the 42nd day after culturing and showed a decreasing pattern thereafter, but it was confirmed that continuous expansion is possible. In addition, it was confirmed that the purity of the NK cells cultured by using the feeder cells of Embodiment 1 was significantly higher than that of the NK cells cultured by using the cells of the control group.

That is, the feeder cells according to an aspect not only significantly increase the activity of NK cells, but also allow long-term culturing of NK cells, so that NK cells may be mass-proliferated and used as a cell therapeutic agent.

### Experimental Example 4. Phenotypic characteristics of NK cells expanded by K562-OX40L-mbIL18-mbIL2 cells

Phenotypic characteristics of NK cells expanded by K562-OX40L-mbIL18-mbIL2 cells were identified. Specifically, after washing the 2 × 10⁵ NK cells, which is derived from a healthy donor (HD) and expanded in Experimental Example 2, with FACS buffer (PBS containing 1 % of FBS), the NK cells were treated with APC-Cyanine7-conjugated mouse anti-human CD3 and PE-Cyanine7-conjugated anti-human CD56 membrane antibodies for 15 minutes. Then, cells were harvested and further stained with different fluorescenceconjugated anti-human CD16, CD69, NKG2D, NKp30, NKp44, NKp46, CD94, CD158a, and CD158b membrane antibodies, for 30 minutes. Thereafter, the cells were washed with FACS buffer, and data were acquired by using a FACS Calibur and analyzed with Kaluza.

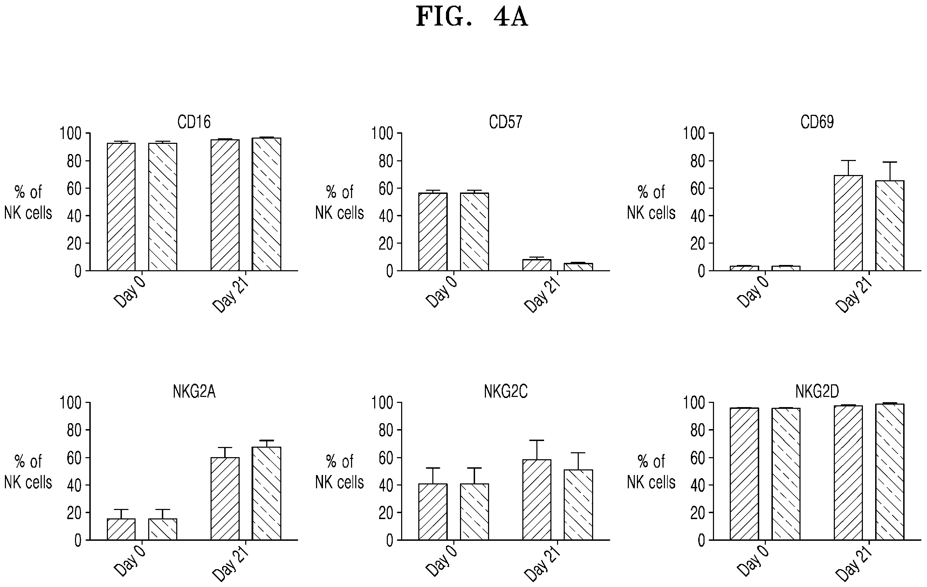

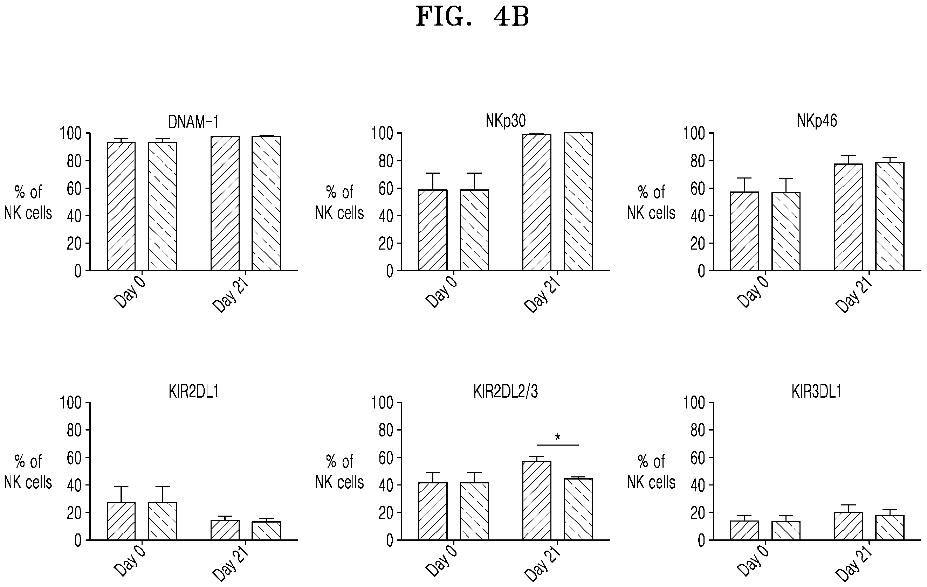

FIGS. 4A and 4B are graphs comparing markers expressed in a healthy donor (HD)-derived NK cells expanded by using the feeder cells according to an aspect.

As a result, as shown in FIG. 4, NK cells expanded by feeder cells expressing OX40L, mbIL18 and mblL21 were confirmed not only to exhibit high NK purity (> 90 %), but also to show increased expression of NK cell activation markers at day 14 compared to the initial stage of the culturing.

That is, it may be seen that the NK cells expanded by the feeder cells according to an aspect have the complete functional characteristics of NK cells.

### Experimental Example 5. Identification of degree of activation of NK cells amplified by K562-OX40L-mbIL18-mbIL2 cells

As expression of the activating receptor increases, activation of the expanded NK cells must also increase, and thus, immunomodulatory activity by cytokine release and cytotoxicity of NK cells was evaluated, wherein the NK cells were derived from cells of a healthy donor HD and expanded by using the feeder cells of Example 1.

### 5-1. Measurement of intracellular IFN-γ

In order to evaluate the immunomodulatory activity, intracellular IFN-γ was measured to evaluate the cytotoxicity of NK cells. Specifically, 2 × 10⁵ NK cells derived from a healthy donor HD expanded by using the feeder cells of Embodiment 1 were cultured in a 96-well round bottom plate in the presence of brefeldin A (BD Biosciences) and Monensin (BD Biosciences) at 37 °C and under the condition of 5 % CO₂ for 5 hours. The cells were then harvested, washed with FACS buffer, and stained with anti-human CD3 and CD56 membrane antibodies for 20 minutes. After washing, fixing and permeabilization, NK cells were further stained with PE-conjugated anti-human IFN-γ antibody on ice for 30 min. Thereafter, the cells were washed and analyzed by using a FACS Calibur flow cytometer.

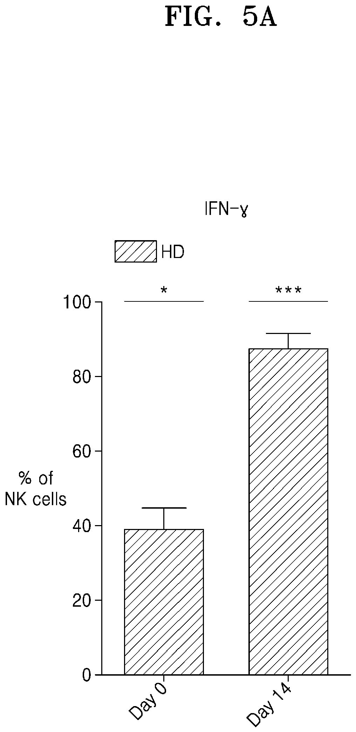

FIG. 5A is a graph confirming cytokines expressed in a healthy donor (HD)-derived NK cells expanded by using the feeder cells according to an aspect.

As a result, as shown in FIG. 5A , it was confirmed that the expression level of IFN-γ in NK cells derived from a healthy donor was significantly increased while the NK cells were being expanded.

### 5-2. Confirmation of degranulation of CD107a and cytotoxicity

The 2 × 10⁵ NK cells derived from a healthy donor HD expanded in Experimental Example 5-1, 2 × 10⁵ target cells (K562, U266, RPMI8226), and PE-conjugated anti-human CD107a were cultured in a 96-well U-bottom plate. After 1 hour, Monensin and brefeldin A (BD Biosciences) were added, and the cells were additionally cultured for 4 hours. Then, NK cells were obtained by staining with anti-human CD3 and CD56 antibodies.

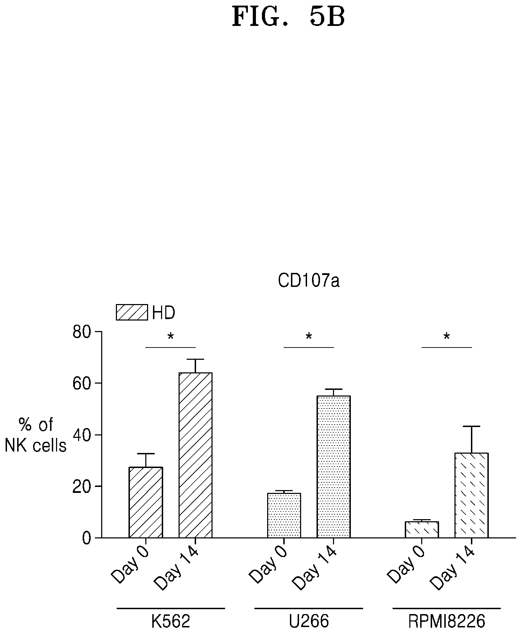

FIG. 5B is a graph showing the results of CD107a degranulation analysis of a healthy donor (HD)-derived NK cells expanded by using the feeder cells according to an aspect.

As a result, as shown in FIG. 5B, after 14 days compared to the initial stage of culturing (day 0), CD107a expression was confirmed to be significantly increased in all the cells (K562, U266, RPMI8226) cultured with healthy donor-derived NK cells.

That is, the results indicate that NK cells with increased activity were successfully generated from peripheral blood mononuclear cells of a healthy donor. Therefore, the feeder cells expressing OX40L, mblL18, and mblL21 according to an embodiment are effective for generating NK cells with increased activity.

### Experimental Example 6. Confirmation of cytotoxicity of NK cells expanded by K562-OX40L-mbIL18-mbIL2 cells

Cytotoxicity was confirmed in order to identify the efficacy of the NK cells expanded by the feeder cells according to an aspect as an antibody therapeutic agent.

Specifically, cytotoxicity of NK cells for target cells on day 14 was measured by a CFSE-based assay for 4 hours. Specifically, target cells were stained with 0.5 µM of carboxyfluorescein succinimidyl ester (CFSE) at 37 °C for 10 minutes in FACS buffer, and washed twice with completed medium. Then, 5 × 10⁴ target cells were placed in a 96-well round bottom plate in triplicate, and the NK cells derived from a healthy donor (HD) and NK cells derived from multiple myeloma (MM) patient were each mixed in an effector-to-target (E: T) ratio of 0.5:1, 1:1 and 2:1. The plate was centrifuged at 1,500 rpm for 3 minutes and then incubated for 4 hours in a 5% CO 2 incubator at 37° C. Thereafter, the mixed cells were then transferred to FACS tubes and 1 µl of 1 mg/mL propidium iodide (PI) (SigmaAldrich, St. Louis, MO, USA) was added to each tube. Cells were then harvested by using a FACS Calibur and analyzed with Kaluza software. The percentage of target cells that died (CFSF-positive and PI-positive) was calculated after subtracting the percentage of target cells that died spontaneously.

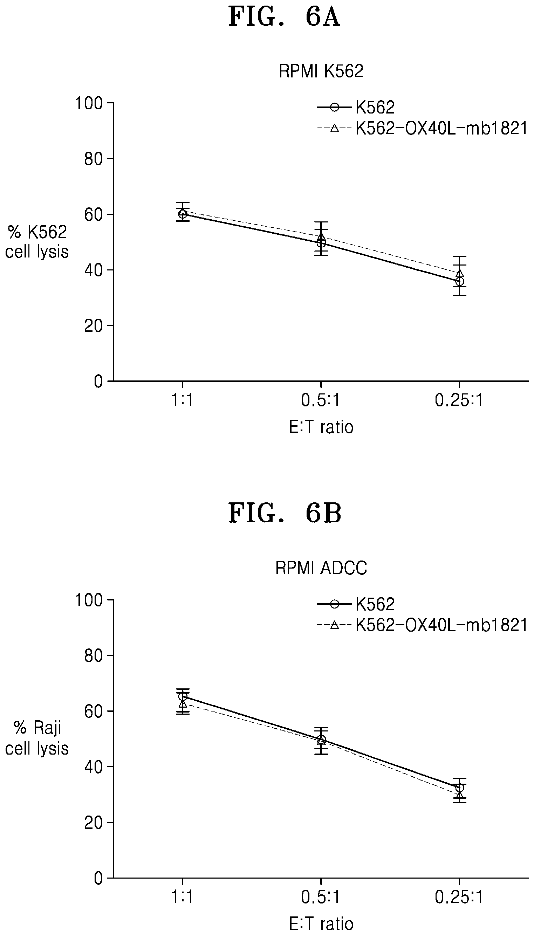

FIG. 6A is a graph confirming the cytotoxicity of a healthy donor (HD)-derived NK cells expanded by using the feeder cells according to an aspect.

FIG. 6B is a graph confirming antibody-dependent cellular cytotoxicity (ADCC) of a healthy donor (HD)-derived NK cells expanded by using the feeder cells according to an aspect.

As a result, as shown in FIG. 6A, the NK cells expanded by using the feeder cells of Example 1 were confirmed to exhibit the same cytotoxicity as the NK cells expanded by using the K562 feeder cells. In addition, as shown in FIG. 4B, the NK cells expanded by using the feeder cells of Embodiment 1 were confirmed to exhibit similar ADCC activity in Raji cells bound to Rituximab as the NK cells expanded by using the K562 feeder cells.

That is, the NK cells expanded by the feeder cells according to an aspect exhibit cytotoxicity, and thus may be utilized as an antibody therapeutic agent.

The above description of the present disclosure is for illustrative purposes, and those with average knowledge in the art to which the present disclosure belongs will be able to understand that the embodiments and examples may be easily modified without changing the technical idea or essential features of the disclosure. Therefore, it should be understood that the above examples are not limitative, but illustrative in all aspects.

## Claims

1. A feeder cell for culturing NK cells, genetically engineered to express membrane bound interleukin-18 (mblL-18), membrane bound interleukin-21 (mbIL-21), and/or OX40L.

2. The feeder cell of claim 1, wherein the feeder cell is selected from the group consisting of K562, RPMI8866, EBV_LCL, 721.221, HFWT, and NK-92 cells.

3. The feeder cell of claim 1, comprising a nucleic acid encoding mbIL-18 and mblL-21.

4. A composition for culturing NK cells, comprising the feeder cell of any one of claims 1 to 3.

5. A method of proliferating NK cells, comprising: obtaining a blood sample containing a population of NK cells; and contacting at least a part of the mixed NK cell population with a genetically engineered cell, wherein the genetically engineered cell is genetically engineered to express membrane bound interleukin-18 (mblL-18), membrane bound interleukin-21 (mbIL-21), and/or OX40L.

6. The method of claim 5, wherein the contacting comprises co-culturing the genetically engineered cell and the mixed NK cell population to expand a subpopulation of the NK cells.

7. The method of claim 6, wherein the co-culturing is performed in the presence of cytokines.

8. The method of claim 7, wherein the cytokines are at least one selected from the group consisting of IL1, IL2, IL3, IL4, IL5, IL6, IL7, IL8 (CXCL8), IL9, IL10, IL11, IL12, IL13, IL14, IL15, IL16, IL17, IL18, IL19, IL20, IL21, IL22, IL23, IL24, IL25, IL26, IL27, IL28, IL29, IL30, IL31, IL32, IL33, IL35, and IL36.

9. The method of claim 8, wherein the cytokines are IL-18 and IL-21.

10. The method of claim 6, wherein the co-culturing is performed for 2 days to 30 days.

11. The method of claim 5, wherein the blood sample is a whole blood sample.

12. The method of claim 5, wherein the genetically engineered cell is treated with radiation of 50 Gy to 300 Gy.
